# EUROPÄISCHE PATENTSCHRIFT

(11) **EP 2 193 118 B1**
(45) Veröffentlichungstag und Bekanntmachung des Hinweises auf die Patenterteilung: **22.08.2012**
(21) Anmeldenummer: 08802402.1
(22) Anmeldetag: 19.09.2008
(51) Int. Cl.: C07D 209/34, C07D 235/26, C07D 249/18, C07D 263/58, C07D 277/68, C07D 413/12, C07D 417/12, C07D 471/10, C07D 491/10, C07D 498/10, A61K 31/404, A61K 31/416, A61K 31/4184, A61K 31/4192, A61K 31/423, A61K 31/428, A61K 31/433, A61P 35/00

(54) **PIPERIDIN- UND PIPERAZINDERIVATE ZUR BEHANDLUNG VON TUMOREN**
PIPERIDINE AND PIPERAZINE DERIVATIVES FOR TREATING TUMOURS
DÉRIVÉS DE PIPÉRIDINE ET DE PIPÉRAZINE POUR LE TRAITEMENT DE TUMEURS

(30) Priorität: 05.10.2007 DE 102007047737
(43) Veröffentlichungstag der Anmeldung: 09.06.2010
(60) Teilanmeldung: 11007349.1
(73) Patentinhaber: Merck Patent GmbH, 64293 Darmstadt (DE)
(72) Erfinder: SCHIEMANN, Kai, 64342 Seeheim-Jugenheim (DE); SCHULTZ, Melanie, 64287 Darmstadt (DE); BLAUKAT, Andree, 69198 Schriesheim (DE); KOBER, Ingo, 64521 Gross-Gerau (DE)
(86) Internationale Anmeldenummer: PCT/EP2008/007893
(87) Internationale Veröffentlichungsnummer: WO 2009/046841

(56) Entgegenhaltungen:
- EP-A1- 0 709 384
- EP-A1- 1 254 661
- WO-A1-00/00197
- WO-A1-02/30422
- WO-A1-98/18793
- WO-A1-99/28318
- WO-A1-02/085352
- JP-A- 60 130 573
- LEIBROCK J ET AL: "EMD 95885, A NEW ELIPRODIL ANALOGUE WITH HIGHER AFFINITY FOR THE N-METHYL-D-ASPARTATE (NMDA) RECEPTOR" PHARMAZIE, DIE, GOVI VERLAG, ESCHBORN, DE, Bd. 52, Nr. 6, 1. Juni 1997 (1997-06-01), Seite 479/480, XP001093620 ISSN: 0031-7144
- YOUS, SAID; LEBEGUE, NICOLAS; POUPAERT, JACQUES-HENRY; CHAVATTE, PHILIPPE; BERTHELOT, PASCAL: "Lipid-lowering properties of 6-benzoyl-2(3H)-benzothiazolone and structurally related compounds" JOURNAL OF ENZYME INHIBITION AND MEDICINAL CHEMISTRY, Bd. 20, 2005, Seiten 525-532, XP008102609
- DATABASE REGISTRY CHEMICAL ABSTRACTS SERVICE, COLUMBUS, OHIO, US; RN 1026456-19-8 XP002516273
- DATABASE REGISTRY CHEMICAL ABSTRACTS SERVICE, COLUMBUS, OHIO, US; RN 732947-29-4 XP002518095

## Beschreibung

### HINTERGRUND DER ERFINDUNG

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen und die Verwendung von Verbindungen zur Behandlung von Krankheiten, die mit einer Erhöhung des Lysophosphatsäure Spiegels einhergehen, ferner pharmazeutische Zusammensetzungen, die diese Verbindungen enthalten.

Im einzelnen betrifft die vorliegende Erfindung Die Verbindungen "B50", "B52" und "B53", die bevorzugt eines oder mehrere Enzyme hemmen, die den Lysophosphatsäure (LPS) Spiegel regulieren und/oder modulieren, Zusammensetzungen, die diese Verbindungen enthalten, sowie Verfahren zu ihrer Verwendung zur Behandlung von Krankheiten und Leiden wie Angiogenese, Krebs, Tumorentstehung, -wachstum und -verbreitung, Arteriosklerose, Augenerkrankungen, choroidale Neovaskularisierung und diabetische Retinopathie, Entzündungserkrankungen, Arthritis, Neurodegeneration, Restenose, Wundheilung oder Transplantatabstossung. Insbesondere eignen sich die erfindungsgemäßen Verbindungen zur Therapie oder Prophylaxe von Krebserkrankungen.

Autotaxin (ATX) ist eine Enzym welches für die Erhöhung des Lysophosphatsäure Spiegel in Ascites und Plasma verantwortlich ist (Xu et al. 1995, Clinical Cancer Research Vol. 1, Seite 1223 und Xu et al. 1995, Biochem. J. Vol- 309, Seite 933). ATX setzt Lysophatidylcholin (LPC) zu Lysophosphatsäure um (Tokumura et al. 2002, J. Biol. Chem., Vol 277, Seite 39436 und Umezu-Gozo et al. 2002, J. Biol. Chem., Vol. 158, Seite 227) LPS ist ein interzellularer Lipid Mediator der eine Vielzahl von biologischen und biochemischen Prozessen wie beispeilsweise glatte Muskelkontraktion, Thrombozyten Aggregation und Apoptose beinflusst (Tigyi et al. 2003 Prog. Lipid Res. Vol 42 , Seite. 498 und Mills et al. 2003 Nat. Rev. Cancer Vol. 3, Seite 582 und Lynch et al. 2001 Prost. Lipid Med. Vol.64, Seite 33). Ausserdem ist LPS in erhöhten Konzentrationen in Plasma und Ascites Flüssigkeit von Ovariar Krebs Patienten der frühen und späten Phase zu finden. LPA spielt dort eine Rolle bei der Tumorzell Proliferation und deren Invasion in benachbarte Gewebe, welche zur Metastasierung führen kann (Xu et al. 1995, Clinical Cancer Research Vol. 1, Seite 1223 und Xu et al. 1995, Biochem. J. Vol- 309, Seite 933). Diese biologischen und phatobiologischen Prozesse werden durch die Aktivierung durch LPA von G-Protein gekoppelten Rezeptoren angeschaltet (Contos et al. 2000, Mol. Pharm. Vol 58, Seite. 1188).

Aus diesem Grunde ist es zur Behandlung von Tumor Patienten wünschenswert, den LPS Spiegel zu senken. Dies kann durch die Hemmung von Enzymen erreicht werden, die an der LPS Biosynthese beteiligt sind, wie beispielsweise Autotaxin (ATX, Sano et al. 2002, J. Biol. Chem. Vol. 277 , Seite 21197 und Aoki et al. 2003, J. Biol. Chem. Vol. 277 Seite 48737). Autotaxin gehört zu der Enzym Familie der Nucleotide Pyrophosphatasen und Phosphodiesterasen (Goding et al. 1998, Immunol. Rev. Vol. 161, Seite 11) und stellt einen wichtigen Ansatzpunkt bei der antitumoralen Therapie dar (Mills et al. 2003 Nat. Rev. Cancer Vol. 3, Seite 582 and Goto eta I. 2004 J. Cell. Biochem. Vol. 92, Seite 1115), da es in Tumoren verstärkte exprimiert wird und Tumorzell Proliferation und deren Invasion in benachbarte Gewebe, welche zur Metastasierung führen kann bewirkt (Nam et al. 2000, Oncogene, Vol. 19 Seite 241). Aussedem beirkt Autotaxin zusammen mit anderen angiogenetischen Faktoren Blugefäßformation im Rahmen der Angiogenese (Nam et al. 2001, Cancer Res. Vol. 61 Seite. 6938). Angiogenes ist eine wichtiger Vorgang beim Tumorwachstum, der die Versorgung des Tumors mit Nährstoffen sichert. Aus diesm Grunde ist die Hemmung der Angiogenes ein wichtiger Ansatzpunkt der Krebs- und Tumortherapie, bie dem der Tumor ausgehungert werden soll (Folkman, 2007, Nature Reviews Drug Discovery Vol. 6, Seite 273-286).

Es wurde überraschend gefunden, daß die erfindungsgemäßen Verbindungen eine spezifische Inhibierung der Enzym Familie der Nucleotide Pyrophosphatasen und Phosphodiesterasen, insbesondere Autotaxin bewirken. Die erfindungsgemäßen Verbindungen zeigen bevorzugt eine vorteilhafte biologische Aktivität, die in den, zum Beispiel hierin beschrieben Assays, leicht nachweisbar ist. In derartigen Assays zeigen und bewirken die erfindungsgemäßen Verbindungen bevorzugt einen inhibierenden Effekt, der gewöhnlich durch IC₅₀-Werte in einem geeigneten Bereich, bevorzugt im mikromolaren Bereich und bevorzugter im nanomolaren Bereich dokumentiert wird.

Generell können alle soliden und nicht soliden Tumore mit den Die Verbindungen "B50", "B52" und "B53" behandelt werden, wie z.B. die Monozytenleukämie, Hirn-, Urogenital-, Lymphsystem-, Magen-, Kehlkopfund Lungenkarzinom, darunter Lungenadenokarzinom und kleinzelliges Lungenkarzinom. Zu weiteren Beispielen zählen Prostata-, Bauchspeicheldrüsen- und Brustkarzinom.

Wie hierin besprochen, sind Wirkungen der erfindungsgemäßen Verbindung für verschiedene Erkrankungen relevant. Dementsprechend sind die erfindungsgemäßen Verbindungen nützlich bei der Prophylaxe und/oder Behandlung von Erkrankungen, die durch eine Inhibierung einer oder mehrerer Nucleotide Pyrophosphatasen und/oder Phosphodiesterasen, insbesondere Autotaxin, beeinflusst werden. Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.

Es kann gezeigt werden, dass die erfindungsgemäßen Verbindungen in einem Xenotransplantat-Tumor-Modell eine vorteilhafte Wirkung aufweisen.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Sensitivität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln zu ermöglichen, Zelltod zu induzieren oder Migration zu inhibieren, gewöhnlich zwischen ungefähr einer Stunde und einer Woche. Zum Testen in vitro können kultivierte Zellen aus einer Biopsieprobe verwendet werden. Die nach der Behandlung zurückbleibenden lebensfähigen Zellen werden dann gezählt.

Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden können.

### STAND DER TECHNIK

Verbindungen, die zur Hemmung von Autotaxin fähig sind, sind in Peng et al. Bioorganic & Medicinal Chemistry (Letters 17, 2007, Seite 1634-1640) beschrieben. Die dort beschriebenen Verbindungen stellen Lipid Analoga dar, welche strukturell keine Gemeinsamkeiten mit den erfindungsgemäßen Verbindungen aufweisen.

Andere heterocyclische Derivate sind beschrieben in WO 2002085352, WO 2002030422, EP 1002535, WO 9818793, EP 385848, FR 2637286, WO 2005097782, EP 709384, EP 396282, EP 49203.

### ZUSAMMENFASSUNG DER ERFINDUNG

Die Erfindung betrifft die Verbindungen "B50", "B52" und "B53" 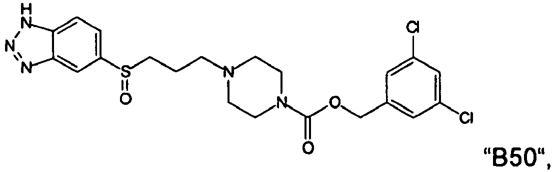 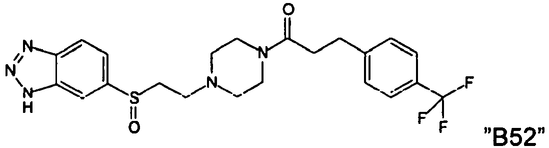 und 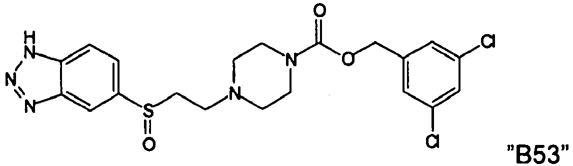 sowie ihre pharmazeutisch verwendbaren Tautomere, Salze und Stereoisomere, einschließlich deren Mischungen in allen Verhältnissen.

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter den Verbindungen "B50", "B52" und "B53" werden auch die Solvate und Derivate verstanden.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.

Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.

Die Bezeichnung "therapeutisch wirksame Menge" umfaßt auch die Mengen, die wirkungsvoll sind, die normale physiologische Funktion zu erhöhen.

Gegenstand der Erfindung ist auch die Verwendung von Mischungen der Verbindungen "B50", "B52" und "B53", z.B. Gemische zweier Diastereomerer z.B. im Verhältnis 1:1, 1:2, 1:3, 1:4, 1:5, 1:10, 1:100 oder 1:1000.

Besonders bevorzugt handelt es sich dabei um Mischungen stereoisomerer Verbindungen.

Die Verbindungen "B50", "B52" und "B53" und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verindungen "B50", "B52" und "B53" umsetzt.

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen "B50", "B52" und "B53" werden größtenteils konventionell hergestellt. Sofern die Verbindungen "B50", "B52" und "B53" eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen "B50", "B52" und "B53" zählen ebenfalls dazu. Bei den Verbindungen "B50", "B52" und "B53" lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen "B50", "B52" und "B53" die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen "B50", "B52" und "B53", die sich von pharmazeutisch unbedenklichen organischen nichttoxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser- als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen "B50", "B52" und "B53" werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen "B50", "B52" und "B53" mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln, im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine der Verbindungen "B50", "B52" und "B53" in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel, enthaltend mindestens eine der Verbindungen "B50", "B52" und "B53" und/oder ihre pharmazeutisch verwendbaren und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder ÖI-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen "B50", "B52" und "B53" sowie Salze und physiologisch funktionelle Derivate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen "B50", "B52" und "B53" sowie die Salze und physiologisch funktionellen Derivate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (Iyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer der Verbindungen "B50", "B52" und "B53" hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per se* bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine Die Verbindungen "B50", "B52" und "B53" und/oder ihre pharmazeutisch verwendbaren und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer der Verbindungen "B50", "B52" und "B53" und/oder ihrer pharmazeutisch verwendbaren und Stereoisomere, einschließlich deren Mischungen in allen Verhältnissen, und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer der Verbindungen "B50", "B52" und "B53" und/oder ihrer pharmazeutisch verwendbaren und Stereoisomere, einschließlich deren Mischungen in allen Verhältnissen, und einer wirksamen Menge eines weiteren Arzneimittelwirkstoffs gelöst oder in Iyophilisierter Form vorliegt.

Bevorzugt aber nicht ausschliesslich werden die Arzneimittel der Tabelle 1 mit den Verbindungen "B50", "B52" und "B53" kombiniert.

| Tabelle 1. | | |
|---|---|---|
| Alkylierungsmittel | Cyclophosphamid | Lomustin |
| | Busulfan | Procarbazin |
| | Ifosfamid | Altretamin |
| | Melphalan | Estramustinphosphat |
| | Hexamethylmelamin | Mechlorethamin |
| | Thiotepa | Streptozocin |
| | Chlorambucil | Temozolomid |
| | Dacarbazin | Semustin |
| | Carmustin | |
| | | |
| Platinmittel | Cisplatin | Carboplatin |
| | Oxaliplatin | ZD-0473 (AnorMED) |
| | Spiroplatin | Lobaplatin (Aetema) |
| | Carboxyphthalatoplatinum | Satraplatin (Johnson Matthey) |
| | Tetraplatin | |
| | Ormiplatin | BBR-3464 (Hoffrnann-La Roche) |
| | Iproplatin | |
| | | SM-11355 (Sumitomo) |
| | | AP-5280 (Access) |
| | | |
| Antimetabolite | Azacytidin | Tomudex |
| | Gemcitabin | Trimetrexate |
| | Capecitabin | Deoxycoformycin |
| | 5-Fluoruracil | Fludarabin |
| | Floxuridin | Pentostatin |
| | 2-Chlordesoxyadenosin | Raltitrexed |
| | 6-Mercaptopurin | Hydroxyhamstoff |
| | 6-Thioguanin | Decitabin (SuperGen) |
| | Cytarabin | Clofarabin (Bioenvision) |
| | 2-Fluordesoxycytidin | Irofulven (MGI Pharrna) |
| | Methotrexat | DMDC (Hoffmann-La Roche) |
| | Idatrexate | |
| | | Ethinylcytidin (Taiho) |
| | | |
| Topoisomerase-Inhibitoren | Amsacrin | Rubitecan (SuperGen) |
| | Epirubicin | Exatecanmesylat (Daiichi) |
| | Etoposid | Quinamed (ChemGenex) |
| | Teniposid oder | Gimatecan (Sigma- Tau) |
| | Mitoxantron | Diflomotecan (Beaufour-Ipsen) |
| | Irinotecan (CPT-11) | |
| | 7-Ethyl-10- | TAS-103 (Taiho) |
| | hydroxycamptothecin | Elsamitrucin (Spectrum) |
| | Topotecan | J-107088 (Merck & Co) |
| | Dexrazoxanet | BNP-1350 (BioNumerik) |
| | (TopoTarget) | CKD-602 (Chong Kun Dang) |
| | Pixantron (Novuspharrna) | |
| | Rebeccamycin-Analogon (Exelixis) BBR-3576 (Novuspharrna) | KW-2170 (Kyowa Hakko) |
| | | |
| Antitumor-Antibiotika | Dactinomycin (Actinomycin D) | Amonafid |
| | | Azonafid |
| | Doxorubicin (Adriamycin) | Anthrapyrazol |
| | Deoxyrubicin | Oxantrazol |
| | Valrubicin | Losoxantron |
| | Daunorubicin (Daunomycin) | Bleomycinsulfat (Blenoxan) |
| | Epirubicin | Bleomycinsäure |
| | Therarubicin | Bleomycin A |
| | Idarubicin | Bleomycin B |
| | Rubidazon | Mitomycin C |
| | Plicamycinp | MEN-10755 (Menarini) |
| | Porfiromycin | GPX-100 (Gem Pharmaceuticals) |
| | Cyanomorpholinodoxorubi cin | |
| | Mitoxantron (Novantron) | |
| | | |
| Antimitotische Mittel | Paclitaxel | SB 408075 |
| | Docetaxel | (GlaxoSmithKline) |
| | Colchicin | E7010 (Abbott) |
| | Vinblastin | PG-TXL (Cell Therapeutics) |
| | Vincristin | |
| | Vinorelbin | IDN 5109 (Bayer) |
| | Vindesin | A 105972 (Abbott) |
| | Dolastatin 10 (NCI) | A 204197 (Abbott) |
| | Rhizoxin (Fujisawa) | LU 223651 (BASF) |
| | Mivobulin (Warner-Lambert) | D 24851 (ASTA Medica) |
| | | ER-86526 (Eisai) |
| | Cemadotin (BASF) | Combretastatin A4 (BMS) |
| | RPR 109881A (Aventis) | Isohomohalichondrin-B (PharmaMar) |
| | TXD 258 (Aventis) | |
| | Epothilon B (Novartis) | ZD 6126 (AstraZeneca) |
| | T 900607 (Tularik) | PEG-Paclitaxel (Enzon) |
| | T 138067 (Tularik) | AZ10992 (Asahi) |
| | Cryptophycin 52 (Eli Lilly) | !DN-5109 (Indena) |
| | Vinflunin (Fabre) | AVLB (Prescient NeuroPharma) |
| | Auristatin PE (Teikoku Hormone) | |
| | | Azaepothilon B (BMS) |
| | BMS 247550 (BMS) | BNP- 7787 (BioNumerik) |
| | BMS 184476 (BMS) | CA-4-Prodrug (OXiGENE) |
| | BMS 188797 (BMS) | Dolastatin-10 (NrH) |
| | Taxoprexin (Protarga) | CA-4 (OXiGENE) |
| Aromatase- Inhibitoren | Aminoglutethimid | Exemestan |
| | Letrozol | Atamestan (BioMedicines) |
| | Anastrazol | YM-511 (Yamanouchi) |
| | Formestan | |
| | | |
| Thymidylatsyntha se-Inhibitoren | Pemetrexed (Eli Lilly) | Nolatrexed (Eximias) |
| | ZD-9331 (BTG) | CoFactor™ (BioKeys) |
| | | |
| DNA- | Trabectedin (PharmaMar) | Mafosfamid (Baxter |
| Antagonisten | Glufosfamid (Baxter | International) |
| | International) | Apaziquon (Spectrum |
| | Albumin + 32P (Isotope | Pharmaceuticals) |
| | Solutions) | 06-Benzylguanin |
| | Thymectacin (NewBiotics) | (Paligent) |
| | Edotreotid (Novartis) | |
| | | |
| Farnesyltransfera se-Inhibitoren | Arglabin (NuOncology Labs) | Tipifarnib (Johnson & Johnson) |
| | Ionafarnib (Schering-Plough) | Perillylalkohol (DOR BioPharma) |
| | BAY-43-9006 (Bayer) | |
| | | |
| Pumpen- | CBT-1 (CBA Pharma) | Zosuquidar-Trihydrochlorid (Eli Lilly) |
| Inhibitoren | Tariquidar (Xenova) | |
| | MS-209 (Schering AG) | Biricodar-Dicitrat (Vertex) |
| | | |
| Histonacetyltransf | Tacedinalin (Pfizer) | Pivaloyloxymethylbutyrat (Titan) |
| erase- | SAHA (Aton Pharma) | |
| Inhibitoren | MS-275 (Schering AG) | Depsipeptid (Fujisawa) |
| | | |
| Metalloproteinase- Inhibitoren Ribonucleosidred uktase-Inhibitoren | Neovastat (Aeterna | CMT -3 (CollaGenex) |
| | Laboratories) | BMS-275291 (Celltech) |
| | Marimastat (British | Tezacitabin (Aventis) |
| | Biotech) | Didox (Molecules for |
| | Galliummaltolat (Titan) | Health) |
| | Triapin (Vion) | |
| | | |
| TNF-alpha-Agonisten/Antago nisten | Virulizin (Lorus Therapeutics) | Revimid (Celgene) |
| | | |
| | CDC-394 (Celgene) | |
| | | |
| Endothelin-A- Rezeptor- Antagonisten | Atrasentan (Abbot) | YM-598 (Yamanouchi) |
| | ZD-4054 (AstraZeneca) | |
| | | |
| Retinsäurerezeptor-Agonisten | Fenretinid (Johnson & Johnson) | Alitretinoin (Ligand) |
| | | |
| | LGD-1550 (Ligand) | |
| | | |
| Immunmodulatore n | Interferon | Dexosom-Therapie (Anosys) |
| | Oncophage (Antigenics) | |
| | GMK (Progenics) | Pentrix (Australian Cancer Technology) |
| | Adenokarzinom-Impfstoff (Biomira) | |
| | | JSF-154 (Tragen) |
| | CTP-37 (AVI BioPharma) | Krebsimpfstoff (Intercell) |
| | JRX-2 (Immuno-Rx) | Norelin (Biostar) |
| | PEP-005 (Peplin Biotech) | BLP-25 (Biomira) |
| | Synchrovax-Impfstoffe (CTL Immuno) | MGV (Progenics) |
| | | !3-Alethin (Dovetail) |
| | Melanom-Impfstoff (CTL Immuno) | CLL-Thera (Vasogen) |
| | p21-RAS-Impfstoff (GemVax) | |
| | | |
| Hormonelle und antihormonelle Mittel | Östrogene | Prednison |
| | konjugierte Östrogene | Methylprednisolon |
| | Ethinylöstradiol | Prednisolon |
| | Chlortrianisen | Aminoglutethimid |
| | Idenestrol | Leuprolid |
| | Hydroxyprogesteroncaproa t | Goserelin |
| | | Leuporelin |
| | Medroxyprogesteron | Bicalutamid |
| | Testosteron | Flutamid |
| | Testosteronpropionat | Octreotid |
| | Fluoxymesteron | Nilutamid |
| | Methyltestosteron | Mitotan |
| | Diethylstilbestrol | P-04 (Novogen) |
| | Megestrol | 2-Methoxyöstradiol (EntreMed) |
| | Tamoxifen | |
| | Toremofin | Arzoxifen (Eli Lilly) |
| | Dexamethason | |
| | | |
| Photodynamische Mittel | Talaporfin (Light Sciences) | Pd-Bacteriopheophorbid |
| | Theralux | (Yeda) |
| | (Theratechnologies) | Lutetium-Texaphyrin |
| | Motexafin-Gadolinium | (Pharmacyclics) |
| | (Pharmacyclics) | Hypericin |
| | | |
| Tyrosinkinase-Inhibitoren | Imatinib (Novartis) | Kahalid F (PharmaMar) |
| | Leflunomid (Sugen/Pharmacia) | CEP- 701 (Cephalon) |
| | | CEP-751 (Cephalon) |
| | ZDI839 (AstraZeneca) | MLN518 (Millenium) |
| | Erlotinib (Oncogene | PKC412 (Novartis) |
| | Science) | Phenoxodiol O |
| | Canertjnib (Pfizer) | Trastuzumab (Genentech) |
| | Squalamin (Genaera) | C225 (ImClone) |
| | SU5416 (Pharmacia) | rhu-Mab (Genentech) |
| | SU6668 (Pharmacia) | MDX-H210 (Medarex) |
| | ZD4190 (AstraZeneca) | 2C4 (Genentech) |
| | ZD6474 (AstraZeneca) | MDX-447 (Medarex) |
| | Vatalanib (Novartis) | ABX-EGF (Abgenix) |
| | PKI166 (Novartis) | IMC-1C11 (ImClone) |
| | GW2016 (GlaxoSmithKline) | |
| | | |
| | EKB-509 (Wyeth) | |
| | EKB-569 (Wyeth) | |
| Verschiedene Mittel | SR-27897 (CCK-A | BCX-1777 (PNP-Inhibitor, |
| | -Inhibitor, Sanofi- | BioCryst) |
| | Synthelabo) | Ranpirnase |
| | Tocladesin (cyclisches- | (Ribonuclease-Stimulans, |
| | AMP-Agonist, Ribapharm) | Alfacell) |
| | Alvocidib (CDK-Inhibitor, | Galarubicin (RNA- |
| | Aventis) | Synthese-Inhibitor, Dong-A) |
| | CV-247 (COX-2-Inhibitor, | |
| | Ivy Medical) | Tirapazamin |
| | P54 (COX-2-Inhibitor, | (Reduktionsmittel, SRI |
| | Phytopharm) | International) |
| | CapCell™ (CYP450- | N-Acetylcystein |
| | Stimulans, Bavarian | (Reduktionsmittel, |
| | Nordic) | Zambon) |
| | GCS-IOO (gal3- | R-Flurbiprofen (NF- |
| | Antagonist, | kappaB-Inhibitor, Encore) |
| | GlycoGenesys) | 3CPA (NF-kappaB- |
| | G17DT-Immunogen | Inhibitor, Active Biotech) |
| | (Gastrin-Inhibitor, Aphton) | Seocalcitol (Vitamin-D- |
| | Efaproxiral (Oxygenator, | Rezeptor-Agonist, Leo) |
| | Allos Therapeutics) | 131-I-TM-601 (DNA-Antagonist, TransMolecular) |
| | PI-88 (Heparanase-Inhibitor, Progen) | |
| | Tesmilifen (Histamin-Antagonist, YM BioSciences) | Eflornithin (ODC-Inhibitor, ILEX Oncology) |
| | | Minodronsäure (Osteoclasten-Inhibitor, Yamanouchi) |
| | Histamin (Histamin-H2-Rezeptor- Agonist, Maxim) | |
| | Tiazofurin (IMPDH-Inhibitor, Ribapharm) | Indisulam (p53-Stimulans, Eisai) |
| | Cilengitid (Integrin-Antagonist, Merck KGaA) | Aplidin (PPT-Inhibitor, PharmaMar) |
| | SR-31747 (IL-1-Antagonist, Sanofi- | Rituximab (CD20-Antikörper, Genentech) |
| | Synthelabo) | Gemtuzumab (CD33- |
| | CCI-779 (mTOR-Kinase- | Antikörper, Wyeth Ayerst) |
| | Inhibitor, Wyeth) | PG2 (Hämatopoese- |
| | Exisulind (PDE-V-Inhibitor, | Verstärker, |
| | Cell Pathways) | Pharmagenesis) |
| | CP-461 (PDE-V-Inhibitor, | Immunol™ (Triclosan- |
| | Cell Pathways) | Oralspülung, Endo) |
| | AG-2037 (GART-Inhibitor,) | Triacetyluridin (Uridin- |
| | Pfizer) | Prodrug, Wellstat) |
| | WX-UK1 | SN-4071 (Sarkom-Mittel, |
| | (Plasminogenaktivator- | Signature BioScience) |
| | Inhibitor, Wilex) | TransMID-107™ |
| | PBI-1402 (PMN-Stimulans, | (Immunotoxin, KS |
| | ProMetic LifeSciences) | Biomedix) |
| | Bortezomib (Proteasom- | PCK-3145 (Apoptose- |
| | Inhibitor, Millennium) | Förderer, Procyon) |
| | SRL-172 (T-Zell-Stimulans, SR Pharma) | Doranidazol (Apoptose-Förderer, Pola) |
| | TLK-286 (Glutathion-S-Transferase-Inhibitor, Telik) | CHS-828 (cytotoxisches Mittel, Leo) |
| | | trans-Retinsäure (Differentiator, NIH) |
| | PT-100 (Wachstumsfaktor-Agonist, Point Therapeutics) | |
| | | MX6 (Apoptose-Förderer, MAXIA) |
| | Midostaurin (PKC-Inhibitor, Novartis) | Apomin (Apoptose-Förderer, ILEX Oncology) |
| | Bryostatin-1 (PKC-Stimulans, GPC Biotech) | Urocidin (Apoptose-Förderer, Bioniche) |
| | CDA-II (Apoptose-Förderer, Everlife) | Ro-31-7453 (Apoptose-Förderer, La Roche) |
| | SDX-101 (Apoptose-Förderer, Salmedix) | Brostallicin (Apoptose-Förderer, Pharmacia) |
| | Ceflatonin (Apoptose-Förderer, ChemGenex) | |
| | | |
| Alkylierungsmittel | Cyclophosphamid | Lomustin |
| | Busulfan | Procarbazin |
| | Ifosfamid | Altretamin |
| | Melphalan | Estramustinphosphat |
| | Hexamethylmelamin | Mechlorethamin |
| | Thiotepa | Streptozocin |
| | Chlorambucil | Temozolomid |
| | Dacarbazin | Semustin |
| | Carmustin | |
| Platinmittel | Cisplatin | Carboplatin |
| | Oxaliplatin | ZD-0473 (AnorMED) |
| | Spiroplatin | Lobaplatin (Aetema) |
| | Carboxyphthalatoplatinum | Satraplatin (Johnson |
| | Tetraplatin | Matthey) |
| | Ormiplatin | BBR-3464 (Hoffrnann-La |
| | Iproplatin | Roche) |
| | | SM-11355 (Sumitomo) |
| | | AP-5280 (Access) |
| | | |
| Antimetabolite | Azacytidin | Tomudex |
| | Gemcitabin | Trimetrexate |
| | Capecitabin | Deoxycoformycin |
| | 5-Fluoruracil | Fludarabin |
| | Floxuridin | Pentostatin |
| | 2-Chlordesoxyadenosin | Raltitrexed |
| | 6-Mercaptopurin | Hydroxyharnstoff |
| | 6-Thioguanin | Decitabin (SuperGen) |
| | Cytarabin | Clofarabin (Bioenvision) |
| | 2-Fluordesoxycytidin | Irofulven (MGI Pharrna) |
| | Methotrexat | DMDC (Hoffmann-La Roche) |
| | Idatrexate | |
| | | Ethinylcytidin (Taiho) |
| | | |
| Topoisomerase-Inhibitoren | Amsacrin | Rubitecan (SuperGen) |
| | Epirubicin | Exatecanmesylat (Daiichi) |
| | Etoposid | Quinamed (ChemGenex) |
| | Teniposid oder | Gimatecan (Sigma- Tau) |
| | Mitoxantron | Diflomotecan (Beaufour-Ipsen) |
| | Irinotecan (CPT-11) | |
| | 7-Ethyl-10- | TAS-103 (Taiho) |
| | hydroxycamptothecin | Elsamitrucin (Spectrum) |
| | Topotecan | J-107088 (Merck & Co) |
| | Dexrazoxanet (TopoTarget) | BNP-1350 (BioNumerik) |
| | | CKD-602 (Chong Kun Dang) |
| | Pixantron (Novuspharrna) | |
| | Rebeccamycin-Analogon (Exelixis) | KW-2170 (Kyowa Hakko) |
| | BBR-3576 (Novuspharrna) | |
| Antitumor-Antibiotika | Dactinomycin (Actinomycin D) | Amonafid |
| | | Azonafid |
| | Doxorubicin (Adriamycin) | Anthrapyrazol |
| | Deoxyrubicin | Oxantrazol |
| | Valrubicin | Losoxantron |
| | Daunorubicin (Daunomycin) | Bleomycinsulfat (Blenoxan) |
| | Epirubicin | Bleomycinsäure |
| | Therarubicin | Bleomycin A |
| | Idarubicin | Bleomycin B |
| | Rubidazon | Mitomycin C |
| | Plicamycinp | MEN-10755 (Menarini) |
| | Porfiromycin | GPX-100 (Gem Pharmaceuticals) |
| | Cyanomorpholinodoxorubi cin | |
| | Mitoxantron (Novantron) | |
| | | |
| Antimitotische Mittel | Paclitaxel | SB 408075 (GlaxoSmithKline) |
| | Docetaxel | |
| | Colchicin | E7010 (Abbott) |
| | Vinblastin | PG-TXL (Cell |
| | Vincristin | Therapeutics) |
| | Vinorelbin | IDN 5109 (Bayer) |
| | Vindesin | A 105972 (Abbott) |
| | Dolastatin 10 (NCI) | A 204197 (Abbott) |
| | Rhizoxin (Fujisawa) | LU 223651 (BASF) |
| | Mivobulin (Warner-Lambert) | D 24851 (ASTA Medica) |
| | | ER-86526 (Eisai) |
| | Cemadotin (BASF) | Combretastatin A4 (BMS) |
| | RPR 109881 A (Aventis) | Isohomohalichondrin-B |
| | TXD 258 (Aventis) | (PharmaMar) |
| | Epothilon B (Novartis) | ZD 6126 (AstraZeneca) |
| | T 900607 (Tularik) | PEG-Paclitaxel (Enzon) |
| | T 138067 (Tularik) | AZ10992 (Asahi) |
| | Cryptophycin 52 (Eli Lilly) | !DN-5109 (Indena) |
| | Vinflunin (Fabre) | AVLB (Prescient |
| | Auristatin PE (Teikoku | NeuroPharma) |
| | Hormone) | Azaepothilon B (BMS) |
| | BMS 247550 (BMS) | BNP- 7787 (BioNumerik) |
| | BMS 184476 (BMS) | CA-4-Prodrug (OXiGENE) |
| | BMS 188797 (BMS) | Dolastatin-10 (NrH) |
| | Taxoprexin (Protarga) | CA-4 (OXiGENE) |
| | | |
| Aromatase-Inhibitoren | Aminoglutethimid | Exemestan |
| | Letrozol | Atamestan (BioMedicines) |
| | Anastrazol | YM-511 (Yamanouchi) |
| | Formestan | |
| Thymidylatsyntha se-Inhibitoren | Pemetrexed (Eli Lilly) | Nolatrexed (Eximias) |
| | ZD-9331 (BTG) | CoFactor™ (BioKeys) |
| | | |
| DNA-Antagonisten | Trabectedin (PharmaMar) | Mafosfamid (Baxter |
| | Glufosfamid (Baxter | International) |
| | International) | Apaziquon (Spectrum |
| | Albumin + 32P (Isotope | Pharmaceuticals) |
| | Solutions) | O6-Benzylguanin |
| | Thymectacin (NewBiotics) | (Paligent) |
| | Edotreotid (Novartis) | |
| | | |
| Farnesyltransfera se-Inhibitoren | Arglabin (NuOncology Labs) | Tipifarnib (Johnson & Johnson) |
| | Ionafarnib (Schering-Plough) | Perillylalkohol (DOR BioPharma) |
| | BAY-43-9006 (Bayer) | |
| | | |
| Pumpen-Inhibitoren | CBT-1 (CBA Pharma) | Zosuquidar-Trihydrochlorid (Eli Lilly) |
| | Tariquidar (Xenova) | |
| | MS-209 (Scherinq AG) | Biricodar-Dicitrat (Vertex) |
| | | |
| Histonacetyltransf erase-Inhibitoren | Tacedinalin (Pfizer) | Pivaloyloxymethylbutyrat (Titan) |
| | SAHA (Aton Pharma) | |
| | MS-275 (Schering AG) | Depsipeptid (Fujisawa) |
| | | |
| Metalloproteinase-Inhibitoren Ribonucleosidred uktase-Inhibitoren | Neovastat (Aeterna | CMT -3 (CollaGenex) |
| | Laboratories) | BMS-275291 (Celltech) |
| | Marimastat (British | Tezacitabin (Aventis) |
| | Biotech) | Didox (Molecules for |
| | Galliummaltolat (Titan) | Health) |
| | Triapin (Vion) | |
| | | |
| TNF-alpha-Agonisten/Antago nisten | Virulizin (Lorus Therapeutics) | Revimid (Celgene) |
| | CDC-394 (Celgene) | |
| | | |
| Endothelin-A-Rezeptor-Antagonisten | Atrasentan (Abbot) | YM-598 (Yamanouchi) |
| | ZD-4054 (AstraZeneca) | |
| | | |
| Retinsäurerezepto r-Agonisten | Fenretinid (Johnson & Johnson) | Alitretinoin (Ligand) |
| | LGD-1550 (Ligand) | |
| Immunmodulatore n | Interferon | Dexosom-Therapie |
| | Oncophage (Antigenics) | (Anosys) |
| | GMK (Progenics) | Pentrix (Australian Cancer |
| | Adenokarzinom-Impfstoff | Technology) |
| | (Biomira) | JSF-154 (Tragen) |
| | CTP-37 (AVI BioPharma) | Krebsimpfstoff (Intercell) |
| | JRX-2 (Immuno-Rx) | Norelin (Biostar) |
| | PEP-005 (Peplin Biotech) | BLP-25 (Biomira) |
| | Synchrovax-Impfstoffe (CTL Immuno) | MGV (Progenics) |
| | | !3-Alethin (Dovetail) |
| | Melanom-Impfstoff (CTL Immuno) | CLL-Thera (Vasogen) |
| | p21-RAS-Impfstoff (GemVax) | |
| | | |
| Hormonelle und antihormonelle Mittel | Ostrogene | Prednison |
| | konjugierte Östrogene | Methylprednisolon |
| | Ethinylöstradiol | Prednisolon |
| | Chlortrianisen | Aminoglutethimid |
| | Idenestrol | Leuprolid |
| | Hydroxyprogesteroncaproa t | Goserelin |
| | | Leuporelin |
| | Medroxyprogesteron | Bicalutamid |
| | Testosteron | Flutamid |
| | Testosteronpropionat | Octreotid |
| | Fluoxymesteron | Nilutamid |
| | Methyltestosteron | Mitotan |
| | Diethylstilbestrol | P-04 (Novogen) |
| | Megestrol | 2-Methoxyöstradiol |
| | Tamoxifen | (EntreMed) |
| | Toremofin | Arzoxifen (Eli Lilly) |
| | Dexamethason | |
| | | |
| Photodynamische Mittel | Talaporfin (Light Sciences) | Pd-Bacteriopheophorbid |
| | Theralux | (Yeda) |
| | (Theratechnologies) | Lutetium-Texaphyrin |
| | Motexafin-Gadolinium | (Pharmacyclics) |
| | (Pharmacyclics) | Hypericin |
| Tyrosinkinase-Inhibitoren | Imatinib (Novartis) | Kahalid F (PharmaMar) |
| | Leflunomid (Sugen/Pharmacia) | CEP- 701 (Cephalon) |
| | | CEP-751 (Cephalon) |
| | ZDI839 (AstraZeneca) | MLN518 (Millenium) |
| | Erlotinib (Oncogene Science) | PKC412 (Novartis) |
| | | Phenoxodiol O |
| | Canertjnib (Pfizer) | Trastuzumab (Genentech) |
| | Squalamin (Genaera) | C225 (ImClone) |
| | SU5416 (Pharmacia) | rhu-Mab (Genentech) |
| | SU6668 (Pharmacia) | MDX-H210 (Medarex) |
| | ZD4190 (AstraZeneca) | 2C4 (Genentech) |
| | ZD6474 (AstraZeneca) | MDX-447 (Medarex) |
| | Vatalanib (Novartis) | ABX-EGF (Abgenix) |
| | PKI166 (Novartis) | IMC-1C11 (ImClone) |
| | GW2016 (GlaxoSmithKline) | |
| | EKB-509 (Wyeth) | |
| | EKB-569 (Wyeth) | |
| | | |
| Verschiedene Mittel | SR-27897 (CCK-A- | BCX-1777 (PNP-Inhibitor, |
| | Inhibitor, Sanofi- | BioCryst) |
| | Synthelabo) | Ranpirnase (Ribonuclease- |
| | Tocladesin (cyclisches- | Stimulans, Alfacell) |
| | AMP-Agonist, Ribapharm) | Galarubicin (RNA- |
| | Alvocidib (CDK-Inhibitor, Aventis) | Synthese-Inhibitor, Dong-A) |
| | CV-247 (COX-2-Inhibitor, | Tirapazamin |
| | Ivy Medical) | (Reduktionsmittel, SRI |
| | P54 (COX-2-Inhibitor, | International) |
| | Phytopharm) | N-Acetylcystein |
| | CapCell™ (CYP450- | (Reduktionsmittel, Zambon) |
| | Stimulans, Bavarian | R-Flurbiprofen (NF- |
| | Nordic) | kappaB-Inhibitor, Encore) |
| | GCS-IOO (gaI3- | 3CPA (NF-kappaB- |
| | Antagonist, GlycoGenesys) | Inhibitor, Active Biotech) |
| | G17DT-Immunogen | Seocalcitol (Vitamin-D- |
| | (Gastrin-Inhibitor, Aphton) | Rezeptor-Agonist, Leo) |
| | Efaproxiral (Oxygenator, | 131-I-TM-601 (DNA- |
| | Allos Therapeutics) | Antagonist, |
| | PI-88 (Heparanase- | TransMolecular) |
| | Inhibitor, Progen) | Eflornithin (ODC-Inhibitor, |
| | Tesmilifen (Histamin- | ILEX Oncology) |
| | Antagonist, YM | Minodronsäure |
| | BioSciences) | (Osteoclasten-Inhibitor, Yamanouchi) |
| | | Indisulam (p53-Stimulans, |
| | Histamin (Histamin-H2- | Eisai) |
| | Rezeptor- Aqonist, | Aplidin (PPT-Inhibitor, |
| | Maxim) | PharmaMar) |
| | Tiazofurin (IMPDH-Inhibitor, Ribapharm) | Rituximab (CD20-Antikörper, Genentech) |
| | Cilengitid (Integrin-Antagonist, Merck KGaA) | Gemtuzumab (CD33-Antikörper, Wyeth Ayerst) |
| | SR-31747 (IL-1-Antagonist, Sanofi-Synthelabo) | PG2 (Hämatopoese-Verstärker, Pharmagenesis) |
| | CCI-779 (mTOR-Kinase-Inhibitor, Wyeth) | Immunol™ (Triclosan-Oralspülung, Endo) |
| | Exisulind (PDE-V-Inhibitor, Cell Pathways) | Triacetyluridin (Uridin-Prodrug, Wellstat) |
| | CP-461 (PDE-V-Inhibitor, Cell Pathways) | SN-4071 (Sarkom-Mittel, Signature BioScience) |
| | AG-2037 (GART-Inhibitor, | TransMID-107™ |
| | Pfizer) | (Immunotoxin, KS |
| | WX-UK1 | Biomedix) |
| | (Plasminogenaktivator- | PCK-3145 (Apoptose- |
| | Inhibitor, Wilex) | Förderer, Procyon) |
| | PBI-1402 (PMN- | Doranidazol (Apoptose-Förderer, Pola) |
| | Stimulans, ProMetic LifeSciences) | CHS-828 (cytotoxisches |
| | Bortezomib (Proteasom- | Mittel, Leo) |
| | Inhibitor, Millennium) | trans-Retinsäure |
| | SRL-172 (T-Zell- | (Differentiator, NIH) |
| | Stimulans, SR Pharma) | MX6 (Apoptose-Förderer, |
| | TLK-286 (Glutathion-S- | MAXIA) |
| | Transferase-Inhibitor, | Apomin (Apoptose- |
| | Telik) | Förderer, ILEX Oncology) |
| | PT-100 (Wachstumsfaktor- | Urocidin (Apoptose- Förderer, Bioniche) |
| | Agonist, Point Therapeutics) | Ro-31-7453 (Apoptose- Förderer, La Roche) |
| | Midostaurin (PKC-Inhibitor, Novartis) | Brostallicin (Apoptose- Förderer, Pharmacia) |
| | Bryostatin-1 (PKC-Stimulans, GPC Biotech) | |
| | CDA-II (Apoptose-Förderer, Everlife) | |
| | SDX-101 (Apoptose-Förderer, Salmedix) | |
| | Ceflatonin (Apoptose-Förderer, ChemGenex) | |

Bevorzugt werden die erfindungsgemäßen Verbindungen mit bekannten Antikrebsmitteln kombiniert:

Zu diesen bekannten Antikrebsmitteln zählen die folgenden: Östrogenrezeptormodulatoren, Androgenrezeptormodulatoren, Retinoidrezeptormodulatoren, Zytotoxika, antiproliferative Mittel, Prenyl-Proteintransferasehemmer, HMG-CoA-Reduktase-Hemmer, HIV-ProteaseHemmer, Reverse-Transkriptase-Hemmer sowie weitere Angiogenese-hemmer. Die vorliegenden Verbindungen eignen sich insbesondere zur gemeinsamen Anwendung mit Radiotherapie. Die synergistischen Wirkungen der Hemmung des VEGF in Kombination mit Radiotherapie sind in der Fachwelt beschrieben worden (siehe WO 00/61186).

"Östrogenrezeptormodulatoren" bezieht sich auf Verbindungen, die die Bindung von Östrogen an den Rezeptor stören oder diese hemmen, und zwar unabhängig davon, wie dies geschieht. Zu den Östrogenrezeptormodulatoren zählen zum Beispiel Tamoxifen, Raloxifen, Idoxifen, LY353381, LY 117081, Toremifen, Fulvestrant, 4-[7-(2,2-Dimethyl-1-oxopropoxy-4-methyl-2-[4-[2-(1-piperidinyl)ethoxy]phenyl]-2H-1-benzopyran-3-yl]phenyl-2,2-dimethylpropanoat, 4,4'-Dihydroxybenzophenon-2,4-dinitrophenylhydrazon und SH646, was jedoch keine Einschränkung darstellen soll.

"Androgenrezeptormodulatoren" bezieht sich auf Verbindungen, die die Bindung von Androgenen an den Rezeptor stören oder diese hemmen, und zwar unabhängig davon, wie dies geschieht. Zu den

Androgenrezeptormodulatoren zählen zum Beispiel Finasterid und andere 5α-Reduktase-Hemmer, Nilutamid, Flutamid, Bicalutamid, Liarozol und Abirateron-acetat.

"Retinoidrezeptormodulatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, α-Difluormethylornithin, ILX23-7553, trans-N-(4'-Hydroxyphenyl)retinamid und N-4-Carboxyphenylretinamid.

"Zytotoxika" bezieht sich auf Verbindungen, die in erster Linie durch direkte Einwirkung auf die Zellfunktion zum Zelltod führen oder die die Zellmyose hemmen oder diese stören, darunter Alkylierungsmittel, Tumornekrosefaktoren, interkaliernde Mittel, Mikrotubulin-Hemmer und Topoisomerase-Hemmer.

Zu den Zytotoxika zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, Irofulven, Dexifosfamid, cis-Amindichlor(2-methylpyridin)platin, Benzylguanin, Glufosfamid, GPX100, (trans,trans,trans)-bis-mu-(hexan-1,6-diamin)-mu-[diamin-platin(II)]bis-[diamin(chlor)platin(II)]-tetrachlorid, Diarizidinylspermin, Arsentrioxid, 1-(11-Dodecylamino-10-hydroxyundecyl)-3,7-dimethylxanthin, Zorubicin, Idarubicin, Daunorubicin, Bisantren, Mitoxantron, Pirarubicin, Pinafid, Valrubicin, Amrubicin, Antineoplaston, 3'-Desamino-3'-morpholino-13-desoxo-10-hydroxycarminomycin, Annamycin, Galarubicin, Elinafid, MEN10755 und 4-Desmethoxy-3-desamino-3-aziridinyl-4-methylsulfonyldaunorubicin (siehe WO 00/50032), was jedoch keine Einschränkung darstellen soll.

Zu den Mikrotubulin-Hemmern zählen zum Beispiel Paclitaxel, Vindesinsulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-dimethyl-L-valyl-L-valyl-N-methyl-L-valyl-L-prolyl-L-prolin-t-butylamid, TDX258 und BMS188797.

Topoisomerase-Hemmer sind zum Beispiel Topotecan, Hycaptamin, Irinotecan, Rubitecan, 6-Ethoxypropionyl-3',4'-O-exo-benzylidenchartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1 H,12H-benzo[de]pyrano[3',4':b,7]indolizino[1,2b]chinolin-10,13(9H,15H)-dion, Lurtotecan, 7-[2-(N-Isopropylamino)ethyl]-(20S)camptothecin, BNP1350, BNPI1100, BN80915, BN80942, Etoposid-phosphat, Teniposid, Sobuzoxan, 2'-Dimethylamino-2'-desoxy-etoposid, GL331, N-[2-(Dimethylamino)ethyl]-9-hydroxy-5,6-dimethyl-6H-pyrido[4,3-b]carbazol-1-carboxamid, Asulacrin, (5a,5aB,8aa,9b)-9-[2-[N-[2-(Dimethylamino)ethyl]-N-methylamino]ethyl]-5-[4-hydroxy-3,5-dimethoxyphenyl]-5,5a,6,8,8a,9-hexohydrofuro(3',4':6,7)naphtho(2,3-d)-1,3-dioxol-6-on, 2,3-(Methylendioxy)-5-methyl-7-hydroxy-8-methoxybenzo[c]phenanthridinium, 6,9-Bis[(2-aminoethyl)amino]benzo[g]isochinolin-5,10-dion, 5-(3-Aminopropylamino)-7,10-dihydroxy-2-(2-hydroxyethylaminomethyl)-6H-pyrazolo[4,5,1-de]-acridin-6-on, N-[1-[2(Diethylamino)ethylamino]-7-methoxy-9-oxo-9H-thioxanthen-4-ylmethyl]formamid, N-(2-(Dimethyl-amino)-ethyl)acridin-4-carboxamid, 6-[[2-(Dimethylamino)-ethyl]amino]-3-hydroxy-7H-indeno[2,1-c]chinolin-7-on und Dimesna.

Zu den "antiproliferativen Mitteln" zählen Antisense-RNA- und -DNA-Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabinocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzofuryl)sulfonyl]-N'-(3,4-dichlorphenyl)harnstoff, N6-[4-Desoxy-4-[N2-[2(E),4(E)-tetradecadienoyl]glycylamino]-L-glycero-B-L-manno-heptopyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Flurouracil, Alanosin, 11-Acetyl-8-(carbamoyloxymethyl)-4-formyl-6-methoxy-14-oxa-1,11-diazatetracyclo-(7.4.1.0.0)-tetradeca-2,4,6-trien-9-ylessigsäureester, Swainsonin, Lometrexol, Dexrazoxan, Methioninase, 2'-cyan-2'-desoxy-N4-palmitoyl-1-B-D-Arabinofuranosylcytosin und 3-Aminopyridin-2-carboxaldehydthiosemicarbazon. Die "antiproliferativen Mittel" beinhalten auch andere monoklonale Antikörper gegen Wachstumsfaktoren als bereits unter den "Angiogenese-Hemmern" angeführt wurden, wie Trastuzumab, sowie Tumorsuppressorgene, wie p53, die über rekombinanten virusvermittelten Gentransfer abgegeben werden können (siehe z.B. US-Patent Nr. 6,069,134).

Offenbart ist die Verwendung der erfindungsgemäßen Verbindung "B50", "B52" und "B53" zur Behandlung und Prophylaxe von Tumorerkrankungen.

Der Tumor ist vorzugsweise ausgewählt aus der Gruppe der Tumoren des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens, des Kehlkopft und/oder der Lunge.

Der Tumor ist weiterhin vorzugsweise ausgewählt aus der Gruppe Lungenadenokarzinom, kleinzellige Lungenkarzinome, Bauchspeicheldrüsenkrebs, Glioblastome, Kolonkarzinom und Brustkarzinom.

Weiterhin offenbart ist die Verwendung zur Behandlung eines Tumors des Blut- und Immunsystems, vorzugsweise zur Behandlung eines Tumors ausgewählt aus der Gruppe der akuten myelotischen Leukämie, der chronischen myelotischen Leukämie, akuten lymphatischen Leukämie und/oder chronischen lymphatischen Leukämie.

Unter einem anderen Aspekt umfasst die Erfindung ein zur Behandlung eines Patienten, der ein Neoplasma, wie einen Krebs, hat, durch Verabreichung einer Verbindung der Formel (I) in Kombination mit einem antiproliferativen Mittel. Geeignete antiproliferative Mittel umfassen die in Tabelle 1 bereitgestellten.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetet oder Dichlormethan, trennt ab, trocknet die organische Phase über Natriumsulfat, dampft ein und reinigt duch Chromatographie an Kieselgel und/oder durch Kristallisation. Rf-Werte an Kieselgel; Laufmittel: Ethylacetat/Methanol 9:1.
- Massenspektrometrie (MS):: EI (Electronenstoß-Ionisation) M⁺
FAB (Fast Atom Bombardment) (M+H)⁺
ESI (Electrospray lonization) (M+H)⁺
APCI-MS (atmospheric pressure chemical ionization - mass spectrometry) (M+H)⁺

### Methode LC/MS:

| | |
|---|---|
| Solvent A: | Wasser + 0,1 % TFA |
| Solvent B: | Acetonitril + 0,1 % TFA |
| Flow: | 2,4 ml/min |
| Gradient: | 0,0 min 4 % B |
| | 2,6 min 100 % B |
| Säule: | Chromolith^{®} Speed ROD RP-18e 50-4, 6 mm |

### Methode HPLC:

| | |
|---|---|
| Solvent A: | Wasser + 0,1 % TFA |
| Solvent B: | Acetonitril + 0,08 % TFA |
| Flow: | 1,5 ml/min |
| Gradient: | 0,0 min 20 % B |
| | 6,0 min 100 % B |
| | 7,0 min 100 % B |
| | 8,0 min 20 % B |
| | 9,0 min 20 % B |
| Säule: | Chromolith^{®} RP18e 100-4, 6 mm |

### Beispiel 1

### Synthese von 4-[2-(3H-Benzotriazol-5-sulfinyl)-ethyl]-piperazin-1-carbonsäure-3,5-dichlorbenzylester ; Hydrochlorid 8

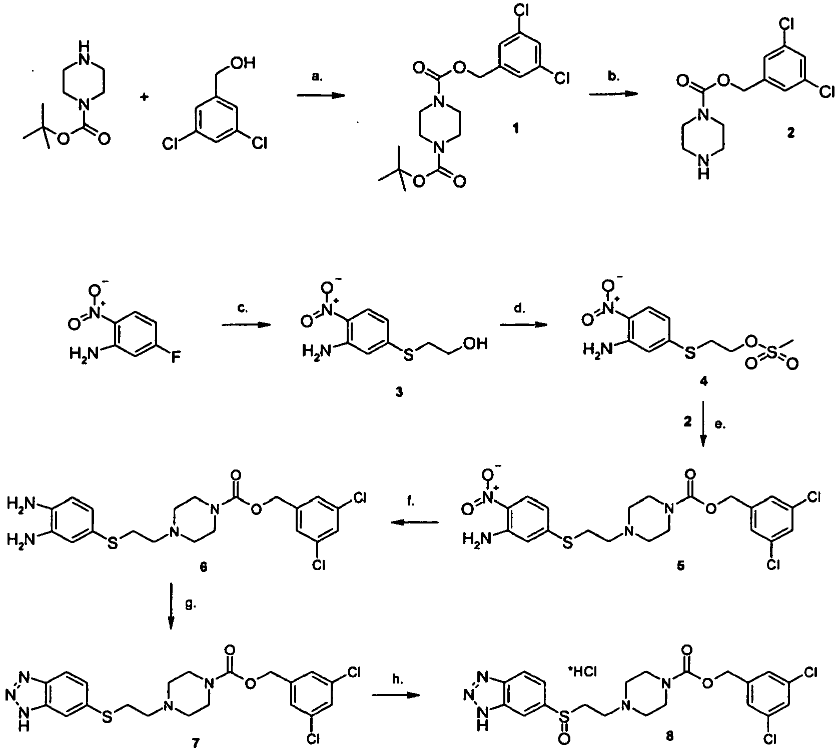

a. 1,1'-Carbonyldiimidazol (5.10g, 31.4 mmol) und (3,5-Dichlorophenyl)-methanol (5.55 g, 31.3 mmol) wird in DMF (70 mL) gelöst und 2h bei RT gerührt. Anschliessend wird Piperazin-1-carbonsäure-tert.-butylester (5.30 g, 28.4 mmol) zugegeben und 18 h bei RT weitergerührt. Das Lösungsmittel wird am Rotationsverdampfer entfernt, mit DCM verdünnt (100 mL) und 2x mit Wasser gewaschen. Die organische Phase wird abgetrennt, über Magnesiumsulfat getrocknet, abfiltriert und zur Trockne eingedampft. Das erhaltene farblose Produkt 1 (10.60 g, 27.2 mmol, 95.6%) wird ohne weitere Aufreinigung umgesetzt.
b. Verbindung 1 (10.60 g, 27.2 mmol) wird in 6N HCl in 2-Propanol (100 mL) aufgenommen und bei RT 2 h gerührt. Das Reaktionsgemisch wird zur Trockene eingeengt. Man erhält einen farblosen Feststoff (10.0 g, 30.7 mmol, 112 %), der sich als die Verbindung 2 herausstellte.
c. 5-Fluor-2-nitroanilin (5.0 g, 32.0 mmol) und 2-Mercaptoethanol (2.23 ml, 32.0 mmol) wird in 50 ml Acetonitril gelöst und auf 80° C erwärmt. Nach 10 Minuten wird Triethylamin (4.44 ml, 32.0 mmol) vorsichtig zugegeben. Es wird 48 h bei 80° C gerührt, wobei noch zweimal 2-Mercaptoethanol (insgesamt 4.46 ml, 64.0 mmol) zugegeben wird. Das Reaktionsgemisch wird eingeengt, mit 200 ml Essigester verdünnt und 2x mit 100 ml Wasser gewaschen. Die organische Phase wird abgetrennt, über Magnesiumsulfat getrocknet, abfiltriert und zur Trockne eingedampft. Das Rohprodukt wird chromatographisch an 150 g Kieselgel aufgereinigt (Laufmittel Petrolether/Essigester 1: 1). Nach Einengen der entsprechenden Fraktionen erhält man ein Öl, welches sich aus Etanol durch Zugabe von Ether kristallisieren läßt. Man erhält 6.9 g (32.2 mmol 100,6%) farblose Kristalle 3.
d. 3 (2,8g, 13.07 mmol) und Trieethylamin (1,80 ml, 13.07 mmol) wird in 40 ml DCM gelöst. Dann wurde Methansulfonylchlorid (1.01 ml, 13.07 mmol) tropfenweise zugegeben. Es wird 2 h gerührt, anschliessend das Reaktionsgemisch eingeengt. Das Rohprodukt 4 (3.8 g, 13.0 mmol, 99.5%) wird nicht weiter aufgereinigt.
e. Die Verbindungen 2 (4.23 g, 13.0 mmol), 4 (3.80 g, 13.0 mmol) und Triethylamin (3.60 ml, 26.0 mmol) werden in 40 ml DMF gelöst und 72 h bei 60° C gerührt. Das Reaktionsgemisch wwird eingeengt, mit 200 ml Essigester verdünnt und 2x mit 100 ml Wasser gewaschen. Die organische Phase wird abgetrennt, über Magnesiumsulfat getrocknet, abfiltriert und zur Trockne eingedampft. Das Rohprodukt wird chromatographisch an 100 g Kieselgel aufgereinigt (Laufmittel Petrolether/Essigester 1:1). Nach Einengen der entsprechenden Fraktionen erhält man 0.73 g (1.50 mmol, 11.6%) 5 als gelbliche Festsubstanz.
f. 5 (0.73 g, 1.50 mmol) werden in 10 ml Tetrahydrofuran gelöst, mit 0.60 g Pd/C (5%) versetzt und bei RT bis zur Vollständigkeit hydriert. Die Versuchsdauer beträg 30 h. Der Katalysator wird abfiltriert. Nach Einengen der Lösung erhält man 0.66 g (1.45 mmol, 96.4%) 6 als Festsubstanz.
g. 0.66 g (1.45 mmol) 6 werden in 20ml Essigsäure gelöst und mit Natriumnitrit (0.10 g, 1.45 mmol) versetzt. Es wird 1 h bei RT gerührt, dann mit Wasser verdünnt und mit 100 ml Essigester extrahiert. Die organische Phase wird mit Magnesiumsulfat getrocknet und eingeengt. Man erhält 0.82 g (Gehalt 82%, 1.44 mmol, 99.3%) 7 als farblose Festsubstanz.
h. 0.82 g (1.44 mmol) 7 werden in 20ml Essigsäure gelöst und mit Wasserstoffperoxid (30%ig) (0.33 ml, 2.90 mmol) versetzt. Es wird 24 h bei RT gerührt, dann mit Wasser verdünnt und mit gesättigter, wässriger NaHCO3-Lösung neutralisiert. Dann wird mit 100 ml Essigester extrahiert. Die organische Phase wird mit Magnesiumsulfat getrocknet und eingeengt. Das Rohprodukt wird chromatographisch an 100 g Kieselgel aufgereinigt (Laufmittel Essigester/Methanol 4: 1). Nach Einengen der entsprechenden Fraktionen wird der Rückstand in 10 ml Methanol gelöst und mit 0.25 ml (1.5 mmol) 6N HCl in Isopropanol versetzt. Nach dem Einengen erhält man 0.2 g (0.39 mmol, 27%) 8 als amorphe Festsubstanz.

Die nachfolgenden Verbindungen können mit dem Fachmann bekannten Methoden hergestellt werden. Vorzugsweise werden sie nach den Synthese- Methoden aus Beispiel 1 hergestellt:

| | | | |
|---|---|---|---|
| "B50" | 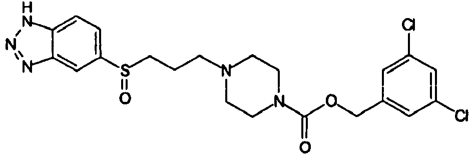 | 497 | 2.72 |
| | 4-[3-(1H-Benzotriazole-5-sulfinyl)-propyl]-piperazin-1-carbonsäure 3,5-dichloro-benzyl ester | | |
| "B52" | 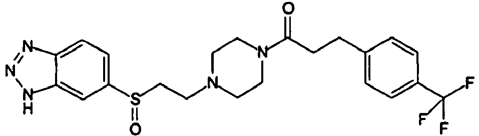 | 481 | 2,64 |
| | 1-{4-[2-(3H-Benzotriazol-5-sulfinyl)-ethyl]-piperazin-1-yl}-3-(4-trifluoromethyl-phenyl)-propan-1-on | | |
| "B53" | 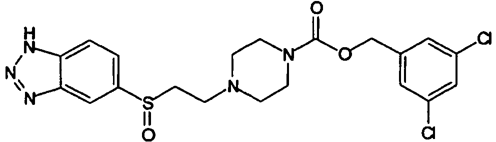 | 483 | 2,91 |
| | 4-[2-(1H-Benzotriazol-5-sulfinyl)-ethyl]-piperazin-1- carbonsäure 3,5-dichloro-benzyl ester | | |

### Beispiel A: Autotaxin Test

### Testbeschreibung

Die Autotaxin Aktivität wird indirekt mit dem Amplex Red Reagenz gemessen. Hierbei wir Amplex Red als fluorgenischem Indikatior für das entstandene H₂O₂ gemessen. Im Detail setzt Autotaxin das Substrat Lysophosphatidylcholin (LPC) zu Phosphocholin und Lysophosphatidyl Säure (LPS) um. Nach dieser Umsetzung wird das Phosphocholin mit alkalischer Phosphatase zu inorganischem Phosphat und Cholin ungesetzt. Im nächsten Schritt wird Cholin durch Choline Oxidase zu Betain oxidiert, wobei H₂O₂ entsteht. H₂O₂ reagiert in Gegenwart von Peroxidase (Horseradish peroxidase) mit dem Amplex Red Reagenz in eine 1:1 Stöchiometrie und bildet das hochfluoreszente Resorufin. Die Fluoreszenz wird in einem reaktionsabhängigen kinetischen Modus gemessen, damit dass fluoreszente Signale möglicher anderer fluoreszenter Stoffe, die nicht an der Reaktion beteiligt sind, herauskorrigiert werden kann.

### Testausführung

1,5 µl einer Standardlösung oder der Testsubstanzen (Substanzen mit dem Namen A(n)) in individuellen Konzentrationen gelöst in 20mM Hepes pH 7.2 mit maximal 7.7% DMSO werden zusammen mit 10 µl (16 ng) hochgereinigten recombinanten Autotaxin in einer schwarzen mit 384 Vertiefungen versehenen Mikrotiterplatte für 30 min bei 22°C vorinkubiert. Danach wird die Reaktion durch Zugabe von 5µl L-α-Lysophosphatidylcholin (LPC) gestartet, wobei die Endkonzentration von LPC 75 µM beträgt. Die Mischung wird 90 min. bei 37°C inkubiert. Nach der Inkubation wird Amplex Red Reagenz, Peroxidase (Horseradish peroxidase) und Cholin Oxidase hinzugefügt und sofort die Fluoreszenz bei 612 nm bei einer Anregung von 485 nm in einem "Tecan Ultra multimode" Lesegerät gemessen. Die Aktivität von Autotaxin wird indirekt über den Nachweis des anfallenden H₂O₂ errechnet.

### Material:

| | |
|---|---|
| Microtiterplatte: | PS-Microplate, 384 Vertiefungen, kleines Volumen, schwarz Corning, Cat#3677 |
| Protein: | Recombinantes Autotaxin (Baculovirale Hi5 Expression) |
| Substrat: | L-α-Lysophosphatidylcholin (Hühnerei)); Avanti Polar Lipids # 830071 P |
| Standard: | C14 LPA, Avanti Polar Lipids, Cat# 857120P |
| Nachweis Reagenz: | Amplex Red Reagenz ; Invitrogen # A12222; gelöst in 1.923 ml of DMSO Peroxidase Type VI-A (horseradish) von Sigma # P6782; gelöst in 7,45 ml Test Puffer,Choline Oxidase ; Sigma # C5896; gelöst in 2,47 ml Test Puffer |
| Nachweis Reagenz Mix: | 1:100 Verdünnung von Amplex Red Regenzt in Test Puffer |
| Test Puffer: | 200 mM Tris-HCl, Merck, Cat # 1.08219, pH 7.9, 0.1 % BSA, lipidfrei, Roche Cat#775835 |

Die nachfolgenden Beispiele betreffen Arzneimittel:

### Beispiel B: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel C: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel D: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel E: Salbe

Man mischt 500 mg eines Wirkstoffes der Formel I mit 99,5 g Vaseline unter aseptischen Bedingungen.

### Beispiel F: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel G: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel H: Kapseln

2 kg Wirkstoff der Formel I werden in üblicher Weise in Hartgelatinekapseln gefüllt, so daß jede Kapsel 20 mg des Wirkstoffs enthält.

### Beispiel I: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen ausgewählt aus der Gruppe
| | |
|---|---|
| "B50" | 4-[3-(1H-Benzotriazole-5-sulfinyl)-propyl]-piperazin-1-carbonsäure 3,5-dichloro-benzyl ester |
| "B52" | 1-{4-[2-(3H-Benzotriazol-5-sulfinyl)-ethyl]-piperazin-1-yl)-3-(4-trifluoromethyl-phenyl)-propan-1-on |
| "B53" | 4-[2-(1 H-Benzotriazol-5-sulfinyl)-ethyl]-piperazin-1-carbonsäure 3,5-dichloro-benzyl ester |
sowie ihre pharmazeutisch verwendbaren Tautomere, Salze und Stereoisomere, einschließlich deren Mischungen in allen Verhältnissen.

2. Arzneimittel, enthaltend mindestens eine Verbindung nach Anspruch 1 und/oder ihre pharmazeutisch verwendbaren, Salze, Tautomere und Stereoisomeren, einschließlich deren Mischungen in allen Verhältnissen, sowie gegebenenfalls Träger- und/oder Hilfsstoffe.

3. Verwendung von Verbindungen nach Anspruch 1 sowie ihrer pharmazeutisch verwendbaren Salze, Tautomeren und Stereoisomeren, einschließlich deren Mischungen in allen Verhältnissen, zur Herstellung eines Arzneimittels.

4. Verwendung von Verbindungen nach Anspruch 1, zur Herstellung eines Arzneimittels zur Behandlung und Prophylaxe von Krebskrankheiten.

5. Verwendung nach Anspruch 4, wobei die Krebskrankheiten mit einem Tumor aus der Gruppe der Tumoren des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens, des Kehlkopfes und/oder der Lunge einhergehen.

6. Verwendung nach Anspruch 5, wobei der Tumor aus der Gruppe Monozytenleukämie, Lungenadenokarzinom, kleinzellige Lungenkarzinome, Bauchspeicheldrüsenkrebs, Glioblastome und Brustkarzinom und Kolokarzinom stammt.

7. Verwendung nach Anspruch 6, wobei die zu behandelnde Krankheit ein Tumor des Blut- und Immunsystems ist.

8. Verwendung nach Anspruch 7, wobei der Tumor aus der Gruppe der akuten myelotischen Leukämie, der chronischen myelotischen Leukämie, akuten lymphatischen Leukämie und/oder chronischen lymphatischen Leukämie stammt.

9. Verwendung von Verbindungen gemäß Anspruch 1 und/oder ihrer physiologisch unbedenklichen Salze und zur Herstellung eines Arzneimittels zur Behandlung von Tumoren wobei eine therapeutisch wirksame Menge einer Verbindung gemäß Anspruch 1 in Kombination mit Radiotherapie und einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird.

## Claims

1. Compounds selected from the group
| | |
|---|---|
| "B50" | 3,5-Dichlorobenzyl 4-[3-(1H-benzotriazole-5-sulfinyl)propyl]-piperazine-1-carboxylate |
| "B52" | 1-{4-[2-(3H-Benzotriazole-5-sulfinyl)ethyl]piperazin-1-yl}-3-(4-trifluoromethylphenyl)propan-1-one |
| "B53" | 3, 5-Dichlorobenzyl 4-[2-(1H-benzotriazole-5-sulfinyl)ethyl]-piperazine-1-carboxylate |
and pharmaceutically usable tautomers, salts and stereoisomers thereof, including mixtures thereof in all ratios.

2. Medicaments comprising at least one compound according to Claim 1 and/or pharmaceutically usable salts, tautomers and stereoisomers thereof, including mixtures thereof in all ratios, and optionally excipients and/or adjuvants.

3. Use of compounds according to Claim 1 and pharmaceutically usable salts, tautomers and stereoisomers thereof, including mixtures thereof in all ratios, for the preparation of a medicament.

4. Use of compounds according to Claim 1 for the preparation of a medicament for the treatment and prophylaxis of cancer diseases.

5. Use according to Claim 4, where the cancer diseases are accompanied with a tumour from the group of the tumours of the squamous epithelium, of the bladder, of the stomach, of the kidneys, of head and neck, of the oesophagus, of the cervix, of the thyroid, of the intestine, of the liver, of the brain, of the prostate, of the urogenital tract, of the lymphatic system, of the stomach, of the larynx and/or of the lung.

6. Use according to Claim 5, where the tumour originates from the group monocytic leukaemia, lung adenocarcinoma, small-cell lung carcinomas, pancreatic cancer, glioblastomas and breast carcinoma and colocarcinoma.

7. Use according to Claim 6, where the disease to be treated is a tumour of the blood and immune system.

8. Use according to Claim 7, where the tumour originates from the group of acute myeloid leukaemia, chronic myeloid leukaemia, acute lymphatic leukaemia and/or chronic lymphatic leukaemia.

9. Use of compounds according to Claim 1 and/or physiologically acceptable salts thereof and for the preparation of a medicament for the treatment of tumours, where a therapeutically effective amount of a compound according to Claim 1 is administered in combination with radiotherapy and a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitor.

## Revendications

1. Composés choisis dans le groupe constitué par
| | |
|---|---|
| "B50" | 4-[3-(1H-benzotriazole-5-sulfinyl)propyl]pipérazine-1-carboxylate de 3,5-dichlorobenzyle |
| "B52" | 1-{4-[2-(3H-benzotriazole-5-sulfinyl)éthyl]pipérazin-1-yl}-3-(4-trifluorométhylphényl)propan-1-one |
| "B53" | 4-[2-(1 H-benzotriazole-5-sulfinyl)éthyl]pipérazine-1-carboxylate de 3,5-dichlorobenzyle |
et les tautomères, sels et stéréoisomères pharmaceutiquement utilisables de ceux-ci, y compris des mélanges de ceux-ci en toutes proportions.

2. Médicaments comprenant au moins un composé selon la revendication 1 et/ou des sels, tautomères et stéréoisomères pharmaceutiquement utilisables de celui-ci, y compris des mélanges de ceux-ci en toutes proportions, et éventuellement des excipients et/ou des adjuvants.

3. Utilisation de composés selon la revendication 1, et de sels, tautomères et stéréoisomères pharmaceutiquement utilisables de ceux-ci, y compris des mélanges de ceux-ci en toutes proportions, pour la préparation d'un médicament.

4. Utilisation de composés selon la revendication 1, pour la préparation d'un médicament destiné au traitement et à la prophylaxie de maladies cancéreuses.

5. Utilisation selon la revendication 4, dans laquelle les maladies cancéreuses sont accompagnées d'une tumeur issue du groupe constitué par les tumeurs de l'épithélium squameux, de la vessie, de l'estomac, des reins, de la tête et du cou, de l'oesophage, du col de l'utérus, de la thyroïde, des intestins, du foie, du cerveau, de la prostate, du tractus urogénital, du système lymphatique, de l'estomac, du larynx et/ou du poumon.

6. Utilisation selon la revendication 5, dans laquelle la tumeur provient du groupe constitué par la leucémie monocytique, l'adénocarcinome du poumon, les carcinomes du poumon à petites cellules, le cancer du pancréas, les glioblastomes, le carcinome du sein et le carcinome du côlon.

7. Utilisation selon la revendication 6, dans laquelle la maladie à traiter est une tumeur du système sanguin et immunitaire.

8. Utilisation selon la revendication 7, dans laquelle la tumeur provient du groupe constitué par la leucémie myéloïde aiguë, la leucémie myéloïde chronique, la leucémie lymphatique aiguë et/ou la leucémie lymphatique chronique.

9. Utilisation de composés selon la revendication 1 et/ou de sels physiologiquement acceptables de ceux-ci, pour la préparation d'un médicament destiné au traitement de tumeurs, dans laquelle une quantité thérapeutiquement efficace d'un composé selon la revendication 1 est administrée en association avec une radiothérapie et un composé issu du groupe : 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) un autre inhibiteur de l'angiogenèse.
